# EUROPEAN PATENT APPLICATION

(11) **EP 4 599 694 A1**
(43) Date of publication of application: **13.08.2025**
(21) Application number: 24184431.5
(22) Date of filing: 25.06.2024
(51) Int. Cl.: A23L 29/10, A23L 29/20, A23L 29/231, A23L 33/115, A61K 9/00, A61K 9/06, A61K 9/107, A61K 47/36

(54) **PECTIN GEL AND PREPARATION METHOD THEREOF**

(30) Priority: 08.02.2024 CN 202410177509
(71) Applicant: Sirio Pharma (Guangdong) Co., Ltd., Shantou Guangdong 515000 (CN)
(72) Inventor: Yu, Ziping, Shantou, Guangdong, 515000 (CN); Huang, Weizhao, Shantou, Guangdong, 515000 (CN)
(74) Representative: Petraz, Gilberto Luigi

(57) **Abstract**

Provided in the present disclosure is a pectin gel and a preparation method thereof, the pectin gel including a gelling agent, an emulsifier and a fat-soluble substance, in which the gelling agent comprises pectin with a degree of esterification of not less than 20%, and, in terms of a mass ratio, the gelling agent:the emulsifier=(0.2-30):1. The degree of esterification (DE) of pectin is the sum of the proportions of methylation, acetylation and amidation in pectin. The degree of esterification of pectin affects the solubility, gelation and emulsification stability of pectin products. The pectin gels provided in the present disclosure allow for improved gelation performance and textural strength, hardness, and elasticity of the pectin gels by limiting the degree of esterification of the pectin and by introducing appropriately proportioned gelling agents and emulsifiers.

## Description

### Field

The present disclosure relates to the field of pectin gels and, particularly, to a pectin gel and a preparation method thereof.

### Background

Pectin is an acidic heteropolysaccharide made of D-galacturonic acid linked by α-1.4-glycosidic bond, which is widely found in the cell walls of higher plants, accounting for about 30%-35%, and it is a natural, green, and healthy natural polymer. Pectin has excellent properties in such as hemostasis, antimicrobial, hypolipidemic, and detoxification, which allows it to be widely used in many fields such as nutraceutical products, food additives, cosmetics, and pharmaceuticals. Pectin regulates the osmotic pressure and pH value of cells, and jelly with a certain hardness and modulus may be formed by adding a certain amount of water. Pectin has been evaluated by JECF to be non-toxic and non-hazardous, and the regulations indicate that the amount of pectin used is not regulated. Therefore, in many countries and regions, pectin is recognized by food legislatures as a food additive with high practical value and non-toxicity, and may be added at the "optimum production requirement" level.

In recent years, pectin has been widely used as a food additive for the preparation of gels containing fat-soluble substances in the food, pharmaceutical, and nutraceutical fields. However, current gel products containing fat-soluble substances have poor gelation properties and are associated with problems such as loose texture. Accordingly, how to prepare a pectin gel containing oil or fat-soluble substances and having better gel properties and texture has become an urgent problem in the field at present.

### Summary

In order to improve the gel properties, texture and structure of a pectin gel containing a fat-soluble substance, provided in the present disclosure is a pectin gel and a preparation method thereof.

In accordance with an aspect of the present disclosure, provided is a pectin gel, including a gelling agent, an emulsifier, and a fat-soluble substance, in which the gelling agent includes pectin with a degree of esterification of not less than 20%, and, in terms of a mass ratio, the gelling agent:the emulsifier=(0.2-30):1. The degree of esterification (hereinafter referred as DE) of pectin is the sum of the proportions of methylation, acetylation, and amidation in pectin. The degree of esterification of pectin affects the solubility, gelation, and emulsification stability of pectin products. The pectin gels provided in the present disclosure allow for improved gelation performance and textural strength, hardness, and elasticity of the pectin gels by limiting the degree of esterification of the pectin and by introducing appropriately proportioned gelling agents and emulsifiers. The mass ratio of the gelling agent to the emulsifier may be, but is not limited to, such as 0.2:1, 0.5:1, 1:1, 1.5:1, 2:1, 3:1, 5:1, 7:1, 10:1, 15:1, 20:1, 25:1 and 30:1, and the degree of esterification of the pectin may be, but is not limited to, such as 20%, 22%, 24%, 26%, 28%, 30%, 32%, 34%, 36%, 38%, 40%, 42%, 44%, 46%, 48%, 50%, 52%, 54%, 56%, 58%, 60%, 62%, 64%, 66%, 68%, 70%, 72%, and 74%, and other values in the range that are not listed are also applicable.

Preferably, the gelling agent includes a first gelling agent and a second gelling agent, the first gelling agent includes pectin, and the second gelling agent includes at least one of gellan gum, locust bean gum, and sodium alginate.

Preferably, the fat-soluble substance includes a fat-soluble physiologically active substance.

Preferably, a mass percentage of the fat-soluble physiologically active substance in the pectin gel is 10%-70%. in which the content of fat-soluble substances in the pectin gel may be, but is not limited to, such as 10%, 12%, 14%, 16%, 18%, 20%, 22%, 24%, 26%, 28%, 30%, 32%, 34%, 36%, 38%, 40%, 42%, 44%, 46%, 48%, 50%, 52%, 54%, 56%, 58%, 60%, 62%, 64%, 66%, 68% and 70%, and other values in the range that are not listed are also applicable.

Preferably, fat-soluble physiologically active substances include at least one of DHA algal oil, oil-soluble vitamins, evening primrose oil, arachidonic acid, flaxseed oil, γ-linolenic acid oil, caprylic/capric triglyceride (MCT), safflower seed oil, silybum marianum seed oil, acer truncatum bunge seed oil, walnut oil, fish oil, coenzyme Q10, rice bran fatty alkanols, pumpkin seed oil, borage oil, acetylsalicylic acid, fat-soluble statins, antibiotics, naproxen, and antihistamine.

Preferably, a water-soluble physiologically active substance.

Preferably, the water-soluble physiologically active substance includes at least one of water-soluble vitamins, water-soluble minerals, ibuprofen, acetaminophen, caffeine, chlorpheniramine maleate, and water-soluble statins.

Preferably, a pH of the pectin gel is 2.6-5. The pH of pectin gels affects the gel properties and texture thereof. The pH of the pectin gel may be, but is not limited to, such as 2.6, 3, 3.5, 4, 4.5, and 5, and other values in the range that are not listed are also applicable.

Preferably, an HLB value of the emulsifier is 7-18. The HLB value of the emulsifier indicates the emulsifying ability of the emulsifier, which affects the dispersion between the gelling agent and the fat-soluble substance. Therefore, it ensures the emulsifying effect when the HLB value of the emulsifier satisfies the range, which improves the homogeneity of the pectin gel, and thus further improves the structural stability of the pectin gel. The HLB value of the emulsifier may be, but is not limited to, such as 7, 8, 9, 10, 11, 12, 13, 14, 15, 16, 17, and 18, and other values in the range that are not listed are also applicable.

Preferably, the emulsifier includes at least one of a phospholipid emulsifier, modified starch, gum, emulsified pectin, cholesterol, lanolin, saponin, and polysaccharide emulsifiers.

Preferably, the phospholipid emulsifier includes at least one of phospholipids, modified phospholipids, and enzymatic phospholipids.

Preferably, the gum includes gum Arabic.

Preferably, the polysaccharide emulsifier includes at least one of polydextrose, polyrhamnose, and polyfuranose.

Preferably, the content of the emulsifier in the pectin gel is 0. 1%-5% in terms of mass percentage, in which the content of the emulsifier may be, but is not limited to, such as 0.1%, 0.2%, 0.3%, 0.50%, 0.75%, 1%, 1.25%, 1.50%, 1.75%, 2%, 2.25%, 2.50%, 3.75%, 3%, 3.25%, 3.50%, 3.75%, 4%, 4.50%, and 5%, and other values in the range that are not listed are also applicable.

Preferably, the degree of esterification of emulsified pectin is greater than 50%. in which the degree of esterification of the emulsified pectin may be, but is not limited to, such as 50%, 52%, 54%, 56%, 58%, 60%, 62%, 64%, 66%, 68%, 70%, 72%, and 74%, and other values in the range that are not listed are also applicable.

Preferably, a content of lipophilic groups in emulsified pectin is 5-35%. in which the content of lipophilic groups in the emulsified pectin may be, but is not limited to, such as 5%, 7%, 9%, 10%, 11%, 13%, 15%, 17%, 19%, 20%, 21%, 23%, 25%, 27%, 29%, 30%, 31%, 33%, and 35%, and other values in the range that are not listed are also applicable.

Preferably, a content of lipophilic groups in emulsified pectin is 15-30%. in which the content of lipophilic groups in the emulsified pectin may be, but is not limited to, such as 15%, 16%, 17%, 18%, 19%, 20%, 21%, 22%, 23%, 24%, 25%, 26%, 27%, 28%, 29%, and 30%, and other values in the range that are not listed are also applicable.

Preferably, lipophilic groups in emulsified pectin include acetyl groups.

Preferably, phospholipid emulsifiers include phospholipids, and the phospholipids include phosphatidylethanolamine (hereinafter referred as PE) and phosphatidylinositol (hereinafter referred as PI). When PE and PI are used together as emulsifiers, it is conducive to the formation of a denser emulsion film, which improves the stability of the emulsion.

Preferably, the sum of the content of phosphatidylethanolamine and phosphatidylinositol in the phospholipid is not less than 10%, in which the sum of the content of phosphatidylethanolamine and phosphatidylinositol in the phospholipid may be, but is not limited to, such as 10%, 12%, 14%, 16%, 18%, 20%, 22%, 24%, 26%, 28%, 30%, 32%, 34%, 36%, 38%, 40%, 42%, 44%, 46%, 48%, 50%, 52%, 54%, 56%, 58%, 60%, 62%, 64%, 66%, 68%, and 70%, and other values in the range that are not listed are also applicable.

Preferably, the sum of the content of phosphatidylethanolamine and phosphatidylinositol in the phospholipid is 30%-70%, in which the sum of the content of phosphatidylethanolamine and phosphatidylinositol in the phospholipid may be, but is not limited to, such as 30%, 32%, 34%, 36%, 38%, 40%, 42%, 44%, 46%, 48%, 50%, 52%, 54%, 56%, 58%, 60%, 62%, 64%, 66%, 68%, and 70%, and other values in the range that are not listed are also applicable.

Preferably, the emulsifier includes a proteinaceous emulsifier, and an isoelectric point of the proteinaceous emulsifier is greater than or equal to 3. The structural stability of the pectin gels is further improved by the use of protein-based emulsifiers with isoelectric point more than or equal to 3, which are able to form anionic polysaccharide-protein conjugates with phytogel combinations to emulsify and stabilize the emulsions.

Preferably, the protein emulsifiers include soy protein and pea protein.

Preferably, the gelling agent includes high-esterified pectin, the high-esterified pectin refers to pectin with a degree of esterification greater than 50%, a content of the high-esterified pectin in the pectin gel is 1.5%-3.5% in terms of mass percentage, and a pH value of the pectin gel is 2.6-3.8. High-esterified pectin is competent to perform gelation under an appropriate degree of esterification and acidic conditions, and when the pH is 2.6-3.8, it affects the number of free radicals in the high-esterified pectin molecule, which consequently affects the gelation properties and texture, in which the content of the high-esterified pectin may be, but is not limited to, such as 1.5%, 1.6%, 1.7%, 1.8%, 1.9%, 2%, 2.1%, 2.2%, 2.3%, 2.4%, 2.5%, 2.6%, 2.7%, 2.8%, 2.9%, 3%, 3.1%, 3.2%, 3.3%, 3.4%, and 3.5%, the pH of the pectin gel may be, but is not limited to, such as 2.6, 2.7, 2.8, 2.9, 3, 3.1, 3.2, 3.3, 3.4, 3.5, 3.6, 3.7, and 3.8, and other values in the range that are not listed are also applicable.

Preferably, the degree of esterification of the high-esterified pectin is not higher than 75%, in which the degree of esterification of the high-esterified pectin may be, but is not limited to, such as 52%, 55%, 57%, 60%, 62%, 65%, 67%, 70%, 72%, and 75%, and other values in the range that are not listed are also applicable.

Preferably, a content of soluble solids of the pectin gel is greater than or equal to 55% in terms of mass percentage. When the gelling agent used for pectin gels is high-esterified pectin, it optimizes the gel stability and emulsification properties of pectin gels when its soluble solids satisfy the range. The content of soluble solids affect the viscosity of the pectin gel system and consequently the emulsification properties, in which the content of soluble solids in the pectin gel may be, but is not limited to, such as 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, and 80%, and other values in the range that are not listed are also applicable.

Preferably, the content of the soluble solids of the pectin gel is 65%-80% in terms of mass percentage, in which the content of the soluble solids of the pectin gel may be, but is not limited to, such as 65%, 67%, 70%, 72%, 75%, 77%, and 80%, and other values in the range that are not listed are also applicable.

Preferably, the pectin gel contains glycerol and sugar alcohol, and a mass of the glycerol:a mass of the sugar alcohol=(1-15):(25-70). On the basis of restricting the content of soluble solids, the ratio of the materials is adjusted by further restricting the sugar level, so that the sugar level in the pectin gel ranges appropriately on the one hand, and on the other hand, the moisturizing effect of the pectin gel is improved, so as to optimize the texture, in which the mass ratio of glycerol to sugar alcohol in pectin gels may be, but is not limited to, such as 1:25, 1:30, 1:35, 1:40, 1:45, 1:50, 1:55, 1:60, 1:65, 1:70, 3:25, 3:30, 3:35, 3:50, 3:60, 3:70, 3:75, 5:25, 5:30, 5:35, 5:50, 5:60, 5:70, 5:75, 10:25, 10: 30, 10:35, 10:50, 10:60, 10:70, 10:75, 15:25, 15:30, 15:35, 15:50, 15:60, 15:70, and 15:75, and other values in the range that are not listed are also applicable.

The content of sugar alcohols in pectin gels is 25%-70%. The presence of a large number of sugar alcohols produces a stable hydrophobic reticulate structure, but the gel performance is decreased after the content of sugar alcohols in the system is greater than 70%, which may be due to the excess of sugar alcohols to make the gel reticulate structure dehydrated, thereby destroying the reticulate structure, in which the content of sugar alcohols in pectin gels may be, but is not limited to, such as 25%, 27%, 29%, 32%, 35%, 37%, 39%, 41%, 40%, 43%, 45%, 47%, 49%, 50%, 51%, 53%, 55%, 57%, 59%, 60%, 61%, 63%, 65%, 67%, 69%, and 70%, and other values in the range that are not listed are also applicable.

Preferably, the sugar alcohol includes at least one of maltitol, maltitol syrup, erythritol, xylitol, sorbitol, and sorbitol liquid.

Preferably, the emulsifier includes phospholipids, and a content of the phospholipids in the pectin gel is 0.1%-5% in terms of mass percentage, in which the content of the phospholipids in the pectin gel may be, but is not limited to, such as 0.1%, 0.3%, 0.5%, 0.7%, 0.9%, 1.1%, 1.3%, 1.5%, 1.7%, 1.9%, 2.1%, 2.3%, 2.5%, 2.7%, 2.9%, 3.1%, 3.3%, 3.5%, 3.7%, 3.9%, 4.1%, 4.3%, 4.5%, 4.7%, and 5.0%, and other values in the range that are not listed are also applicable.

Preferably, the emulsifier includes modified starch, and a content of the modified starch in the pectin gel is 0.5%-4% in terms of mass percentage. By using a certain amount of modified starch as an emulsifier, based on the gelatinization and viscosity-enhancing properties of modified starch, the technical effect is considered from the perspective of emulsification effect and slurry viscosity, in which the viscosity of the slurry also constitutes a certain influence on the gelling speed and gel stability of the product, in which the content of the modified starch in the pectin gel may be, but is not limited to, such as 0.5%, 0.6%, 0.7%, 0.8%, 0.9%, 1%, 1.1%, 1.2%, 1.3%, 1.40%, 1.5%, 1.6%, 1.7%, 1.8%, 1.9%, 2%, 2.1%, 2.2%, 2.3%, 2.4%, 2.5%, 2.5%, 2.6%, 2.7%, 2.8%, 2.9%, 3%, 3.1%, 3.2%, 3.3%, 3.4%, 3.5%, 3.6%, 3.7%, 3.8%, 3.9%, and 4%, and other values in the range that are not listed are also applicable.

Preferably, the emulsifier includes gum Arabic, and a mass of the gum Arabic: a mass of the high-esterified pectin=(0.5-1.5):(1.5-3). Since gum Arabic also belongs to one of the plant gelling agents, the technical effect of simultaneously optimizing the emulsifying properties (corresponding to the homogeneity of the pectin gel) and the gelling properties (corresponding to the textural properties of the pectin gel) was achieved by restricting the ratio of gum Arabic to the high-esterified pectin, in which the mass ratio of gum Arabic to the high-esterified pectin may be, but is not limited to, such as 0.5:2, 0.5:2.5, 0.5:3, 1:2, 1:2.5, 1:3, 1.5:1.5, 1.5:2, 1.5:2.5, 2:1.5, 2:2.5, and 2:3, and other values in the range that are not listed are also applicable.

Preferably, the emulsifier includes modified starch, gum Arabic, in which gum Arabic: modified starch = (0.1-4):1 in terms of mass ratio, in which the mass ratio of gum Arabic to the modified starch may be, but is not limited to, such as 0.1:1, 0.5:1, 0.8:1, 1:1, 1.2:1, 1.5:1, 1.8:1, 2:1, 2.2:1, 2.5:1, 2.8:1, 3:1, 3.2:1, 3.5:1, 3.8:1, and 4:1, and other values in the range that are not listed are also applicable.

Preferably, the pectin includes low-esterified pectin, the low-esterified pectin refers to pectin with a degree of esterification of 20%-50%, a content of the low-esterified pectin in the pectin gel is 1.5%-4% in terms of mass percentage, and a pH value of the pectin gel is 2.6-5, in which the degree of esterification of the low-esterified pectin may be, but is not limited to, such as 20%, 22%, 24%, 26%, 28%, 30%, 32%, 34%, 36%, 38%, 40%, 42%, 44%, 46%, 48%, and 50%, the content of the low-esterified pectin may be, but is not limited to, such as 1.5%, 1.7%, 1.9%, 2.1%, 2.3%, 2.5%, 2.7%, 2.9%, 3.1%, 3.3%, 3.5%, 3.7%, and 4.0%, the pH value of the pectin gel may be, but is not limited to, such as 2.6, 2.8, 3, 3.2, 3.4, 3.6, 3.8, 4, 4.2, 4.4, 4.6, 4.8, and 5, and other values in the range that are not listed are also applicable.

Preferably, a content of soluble solids of the pectin gel is greater than or equal to 10% in terms of mass percentage. When the gelling agent used for pectin gels is low-esterified pectin, the pectin gels provide good gelling properties when the content of the soluble solids satisfies the range, in which, when the gelling agent used for pectin gels is low-esterified pectin, the content of the soluble solids in the pectin gels may be, but is not limited to, such as 10%, 12%, 14%, 16%, 18%, 20%, 22%, 24%, 26%, 28%, 30%, 32%, 34%, 36%, 38%, 40%, 42%, 44%, 46%, 48%, 50%, 52%, 54%, 56%, 58%, 60%, 62%, 64%, 66%, 68%, 70%, 72%, 74 76%, 76%, 78%, and 80, and other values in the range that are not listed are also applicable.

Preferably, the content of the soluble solids in the pectin gels is 50%-80% in terms of mass percentage, in which the content of the soluble solids in the pectin gels may be, but is not limited to, 50%, 52%, 54%, 56%, 58%, 60%, 63%, 65%, 67%, 70%, 72%, 75%, 77%, and 80%, and other values in the range that are not listed are also applicable.

Preferably, the low-esterified pectin includes amide pectin, and a degree of amidation of the amide pectin is 12%-25%. The amide group helps to form hydrogen bonds, so it improves the gelling ability of low-esterified pectin, which is still able to gel under high calcium-ion concentration and high pH value, not easy to be dehydrated, and also allows the gel to have a very good flavor release ability, in which the degree of amidation of the amide pectin may be, but is not limited to, such as 12%, 14%, 16%, 18%, 20%, 22%, 24%, and 25%, and other values in the range that are not listed are also applicable.

Preferably, the pectin gel further includes metallic ions, and a content of the metallic ions in the pectin gel is 0.02-0.25% in terms of mass percentage, in which the content of the metallic ions may be, but is not limited to, such as 0.02%, 0.05%, 0.08%, 0.1%, 0.11%, 0.12%, 0.13%, 0.14%, 0.15%, 0.16%, 0.17%, 0.18%, 0.19%, 0.20%, 0.21%, 0.23%, 0.24%, and 0.25%, and other values in the range that are not listed are also applicable.

Preferably, the metallic ions include at least one of calcium ions and magnesium ions.

Preferably, the calcium ions are provided by a calcium salt, the calcium salt including at least one of calcium lactate, calcium citrate, tricalcium phosphate, calcium hydrogen phosphate, milk mineral salts, calcium gluconate, and calcium chloride.

Preferably, the metallic ions include the calcium ions, a mass percentage of the emulsifier in the pectin gel is a, a mass percentage of the calcium ions in the pectin gel is b, and the pectin gel satisfies 0.4≤a/b≤250. When the mass content of emulsifier and calcium ions satisfies the range, low-esterified pectin is more likely to form an organized cross-linking structure, thereby improving the gel properties and texture of the pectin gels, in which the a!b may be, but is not limited to, such as 0.4, 0.8, 1, 1.5, 3, 5, 10, 20, 40, 60, 80, 100, 120, 140, 160, 180, 200, 220, and 250, and other values in the range that are not listed are also applicable.

Preferably, the pectin gel satisfies 4≤a/b≤150, in which the a!b may be, but is not limited to, such as 4, 7, 10, 20, 40, 60, 80, 100, 120, 140, and 150, and other values in the range that are not listed are also applicable.

Preferably, the content of the emulsifier of the pectin gel is 0.2%-5%, in which the content of the emulsifier may be, but is not limited to, such as 0.2%, 0.5%, 0.8%, 1.1%, 1.4%, 1.7%, 2%, 2.3%, 2.6%, 3%, 3.3%, 3.6%, 3.9%, 4.3%, 4.6%, and 5.0%, and other values in the range that are not listed are also applicable.

Preferably, the pectin gel further includes amino acid, and a content of the amino acid in the pectin gel is no more than 10% in terms of mass percentage, in which the content of the amino acid in the pectin gel may be, but is not limited to, such as 0.5%, 1%, 1.5%, 2%, 3%, 3.5%, 4%, 4.5%, 5%, 5.5%, 6%, 6.5%, 7%, 7.5%, 8%, 8.5%, 9%, 9.5%, and 10%, and other values in the range that are not listed are also applicable.

Preferably, the amino acid includes at least one of γ-aminobutyric acid, creatine, and the like.

Preferably, the pectin gel further includes an antioxidant.

Preferably, the antioxidant includes at least one of tea polyphenols (TP), tocopherols, flavonoids (e.g., rosemary extract), butylhydroxyanisole (BHA), dibutylhydroxytoluene (BHT), tert-butylhydroquinone (TBHQ), and ascorbic acid species.

Preferably, the pectin gel further includes an additive, the additive including at least one of flavor, color, and sweetener.

In accordance with a second aspect of the present disclosure, provided is a preparation method of the aforementioned pectin gel, in which the method includes following steps: preparation of an aqueous phase: dissolving a gelling agent in water in terms of a mass ratio of the gelling agent: water=1:(3-20), and keeping a temperature of a reaction system above 90 °C, and controlling a pH at 3.5-4.3 to obtain the aqueous phase; emulsifying homogenization: in terms of a mass ratio of an oil phase: the aqueous phase = 1: (0.4-9), adding the oil phase to the aqueous phase and mixing homogeneously, emulsifying and homogenizing at 70 °C or above, in which the oil phase includes fat-soluble substances; blending: adding remaining raw materials to emulsified and homogenized liquid to obtain a resulting liquid as a molding liquid; molding: solidifying and molding the molding liquid to obtain the pectin gel.

In the preparation of the aqueous phase, the mass ratio of the gelling agent to the water may be, but is not limited to, such as 1:3, 1:4, 1:5, 1:6, 1:7, 1:8, 1:9, 1:10, 1:11, 1:12, 1:13, 1:14, 1:15, 1:16, 1:17, 1:18, 1:19, and 1:20, the pH value of the aqueous phase may be, but is not limited to, such as 3.5, 3.6, 3.7, 3.8, 3.9, 4, 4.1, 4.2, and 4.3, the mass ratio of the oil phase to the aqueous phase may be, but is not limited to, such as 1:0.4, 1:1, 1:1.5, 1:2, 1:2.5, 1:3, 1:3.5, 1:4, 1:4.5, 1:5, 1:5.5, 1:6, 1:6.5, 1:7, 1:7.5, 1:8, 1:8.5, and 1:9, the temperature of the emulsifying homogenization may be, but is not limited to, such as 70 °C, 72 °C, 74 °C, 76 °C, 78 °C, 80 °C, 82 °C, 84 °C, 86 °C, 88 °C, 90 °C, 92 °C, 94 °C, 96 °C, and 98 °C, and other values in the range that are not listed are also applicable.

Preferably, after the step of emulsifying homogenization is completed, adding an acidity regulator, keeping the temperature of the reaction system above 60 °C, and controlling the pH at 2.6-5. After the step of emulsifying homogenization is completed, the temperature of the reaction system may be, but is not limited to, such as 60 °C, 61 °C, 62 °C, 63 °C, 64 °C, 65 °C, 66 °C, 67 °C, 68 °C, 69 °C, 70 °C, 71 °C, 72 °C, 73 °C, 74 °C, 75 °C, 76 °C, 77 °C, 78 °C, 79 °C, 80 °C, 81 °C, 82 °C, 83 °C, 84 °C, 85 °C, 86 °C, 87 °C, 88 °C, 89 °C, 90 °C, 91 °C, 92 °C, 93 °C, 94 °C, 95 °C, 96 °C, 97 °C, and 98 °C, the pH value may be, but is not limited to, such as 2.6, 2.8, 3, 3.2, 3.4, 3.6, 3.8, 4, 4.2, 4.4, 4.6, 4.8, and 5, and other values in the range that are not listed are also applicable.

Preferably, the acidity regulator includes at least one of citric acid, sodium citrate, potassium citrate, malic acid and lactic acid.

Preferably, the pectin includes low-esterified pectin, and, after the step of emulsifying homogenization is completed, the inorganic salt and acidity regulator are added sequentially, the soluble solids of the reaction system are maintained at more than 30%, the temperature is maintained at more than 60 °C, and the pH is controlled at 2.6-5.

The pectin gel prepared by the preparation method provided in the present disclosure is able to provide the pectin gel with a good texture and further improve the storage stability of the pectin gel, in addition to improving its gelation properties. In addition, by controlling the reaction conditions in the reaction system, such as the reaction temperature, pH, the content of soluble solids and other operations, on the one hand, it is possible to further improve the water-holding property of the pectin gel, and also control the growth and propagation of microorganisms in order to enhance the stability of the food safety; on the other hand, it also maintains the physiological activity of the water-soluble physiologically active substance or the fat-soluble physiologically active substance and ensures that the pectin gel disintegrates quickly when it reaches the proper part of the human body, and such effect facilitates the release of the water-soluble physiologically active substance or the fat-soluble physiologically active substance.

In accordance with a third aspect of the present disclosure, provided is a gel product, including the aforementioned pectin gel, in which a dosage form of the gel product includes at least one of a gummy candy, an oil gel, a gel emulsion, a softgel capsule especially a chewable softgel capsule, a crystal ball, a popping bead, and a drink.

In accordance with a fourth aspect of the present disclosure, provided is an application of the aforementioned pectin gels in food and/or nutraceuticals and/or pharmaceuticals. The food includes general food and/or nutraceutical food.

### Detailed description of the embodiments

For a better understanding of the technical solutions of the present disclosure by those skilled in the art, the technical solutions in the examples of the present disclosure are clearly and completely described and discussed below. Obviously, the examples described herein are only some of the examples of the present disclosure but not all of them. Based on the embodiments in the present disclosure, all other embodiments obtained by those skilled in the art without creative efforts fall within the scope of protection of the present disclosure.

### Example 1

### Processing Group 1A

### 1. Preparation of raw materials for pectin gels

The raw materials required for the preparation of pectin gels for Processing Group 1A of the present example are shown in Table 1. The sum of the content of the phospholipids PE and PI used was 60%.

**Table 1 Raw materials required for the preparation of pectin gels in Processing Group 1A of the present example**

| Components | | Material | Content in percentage/% |
|---|---|---|---|
| oil phase | fat-soluble physiologically active substances | DHA algae oil | 25 |
| | emulsifier | phospholipid | 2 |
| | antioxidant | Rosemary Extract | 0.5 |
| aqueous phase | gelling agent | high-esterified pectin (DE=60%) | 2 |
| | / | sodium citrate | 0.6 |
| | / | glycerol | 10 |
| | | maltitol syrup | 38 |
| | / | water | remnant |
| blending components | acidity regulator | citric acid | 0.9 |

### 2. Preparation method

Preparation of an aqueous phase: weighing the raw materials according to Table 1, adding the gelling agent to water, adding subsequently glycerol, maltitol syrup, and sodium citrate thereto, keeping the temperature of the reaction system above 90 °C, controlling the pH at 4.2, holding the temperature at 85 °C, and obtaining the aqueous phase;
Preparation of an oil phase: weighing the formulated fat-soluble physiological active substances, phospholipids, and antioxidants, mixing them well, and filling with nitrogen pending use;
Emulsifying homogenization: the oil phase: the aqueous phase = 1:3 in terms of a mass ratio, adding the oil phase to the aqueous phase and mixing homogeneously, emulsifying and homogenizing at 80 °C or above;
Blending: adding acidity regulator to the emulsified homogenized liquid, keeping the temperature of the reaction system above 60°C, controlling the pH at 3.5, filling with nitrogen pending use, and using the resulting liquid as a molding liquid;
Molding: pouring the molding liquid onto the blister, sealing, and solidifying to obtain the pectin gel, in which the pH of the pectin gel was 3.5.

Different Processing Groups were set up by constructing variables with the mass ratio between the gelling agent and the emulsifier in the composition of the formulations shown in Table 1, and the corresponding variables for each Processing Group and the Contrasting Group are shown in Table 2, in which the total mass content of the gelling agent and the emulsifier was kept unchanged, and the sum of the masses of the phosphatidylethanolamine and the phosphatidylinositol accounted for 60% of the total mass of the phospholipids. Except for the above differences, the procedure for preparing the pectin gels in each Processing Group was strictly consistent with that of Processing Group 1A of Example 1.

**Table 2 Variables in Processing Groups 2A-4A**

| Groups | gelling agent: emulsifier | mass content of gelling agent | mass content of emulsifier |
|---|---|---|---|
| Processing Group 1A | 1:1 | 2% | 2% |
| Processing Group 2A | 3:1 | 3% | 1% |
| Processing Group 3A | 5:1 | 3.4% | 0.6% |
| Processing Group 4A | 10:1 | 3.64% | 0.36% |
| Contrast Group 1A | 1:5 | 0.6% | 3.4% |

Different Processing Groups were set up using the pH of the pectin gel as a variable, and the corresponding variables for each Processing Group are shown in Table 3. The pH of the pectin gels was achieved by regulating the pH adjusters added during preparation. Except for the above differences, the procedure for preparing the pectin gels in each Processing Group was strictly consistent with that of Processing Group 1A of Example 1.

**Table 3 Variables in Processing Groups 5A-7A**

| Groups | pH of the pectin gel |
|---|---|
| Processing Group 1A | 3.5 |
| Processing Group 5A | 3.8 |
| Processing Group 6A | 3.0 |
| Processing Group 7A | 2.6 |

Different Processing Groups were set up using the composition of emulsifiers (type of phospholipids) in the composition of the formulations shown in Table 1 as variables, and the corresponding variables for each Processing Group are shown in Table 4, in which the total mass percentage of phospholipids in the pectin gels was kept unchanged, and the change in the sum of the contents of PE and PI in the phospholipids was achieved by selecting different specifications of phospholipid emulsifiers. Except for the above differences, the procedure for preparing the pectin gels in each Processing Group was strictly consistent with that of Processing Group 1A of Example 1.

**Table 4 Variables in Processing Groups 8A-10A**

| Groups | Sum/% of PE and PI in phospholipids |
|---|---|
| Processing Group 1A | 60 |
| Processing Group 8A | 30 |
| Processing Group 9A | 45 |
| Processing Group 10A | 70 |

### Testing Example 1

### 1. Test Objects

Pectin gels prepared from each Processing Group and the Contrast Group in Example 1.

### 2. Test Methods

(1) Texture test: The texture test was carried out using a texture meter, using the TPA method (texture profile analysis), which was a common method for testing texture characteristics in the field, and was also known as the secondary mastication test, in which the samples were compressed for a second time through the test probe, and then quantified by the force sensing element for the index. The samples were taken and placed in the middle of the test bench, the TPA method was selected, and the test key was activated for testing, in which the control samples were of uniform specifications and shapes, pancake shaped, 1.5g/piece, with a weight deviation of no more than 0.1g. 20 samples were tested in parallel in each group, and the average value was taken as the result of the texture test.
   When the test results of hardness <500g and resilience <0.24, it was recorded as unqualified; when the test results of hardness ranging 500g-1500g and resilience ranging 0.24-0.30, it was recorded as qualified, and when the test results of hardness ≥1500g and resilience ≥0.30, it was recorded as outstanding.
(2) Storage stability: placing the test object under the environmental conditions at 50 °C and 80% relative humidity for 24h and observing the deformation of the test object, in which the presence of floppy deformation was recorded as unqualified, the absence of obvious floppy deformation was recorded as qualified, and the absence of floppy deformation was recorded as outstanding.

### 3. Test results and analysis

The results of the present Testing Example are shown in Table 5, in which it was evident from comparing the data of the Processing Groups 1A-4A and the Contrast Group 1A, that the ratio of the gelling agent to the emulsifier affected the mechanical properties, texture, and storage stability of the prepared pectin gels. By further preference of the present disclosure, the ratio of the gelling agent to the emulsifier satisfied (0.2-30):1, and, when the range of the gelling agent satisfied 1.5%-4%, the storage stability of the pectin gel was enhanced. In Processing Group 1A and Processing Groups 5A-7A, it was confirmed that the pH of the pectin gels also affected the mechanical properties, texture, and storage stability of the prepared pectin gels, and that the pectin gels provided a better texture and storage stability when the pH of the pectin gels ranged from 2.6 to 3.8. In Processing Group 1A, and Processing Groups 8A-10A, it was confirmed that the sum of the content of PE and PI in the phospholipids likewise affected the mechanical properties, texture, and storage stability of the prepared pectin gels.

Therefore, an improvement in the texture and storage stability of a pectin gel containing a fat-soluble substance was achieved in the present disclosure by regulating the ratio of a gelling agent to an emulsifier in the pectin gel, the pH of the pectin gel, and the sum of the content of PE and PI in the phospholipid.

**Table 5 Test results of Testing Example 1**

| Groups | Texture Testing | Storage Stability |
|---|---|---|
| Processing Group 1A | outstanding | outstanding |
| Processing Group 2A | outstanding | outstanding |
| Processing Group 3A | outstanding | qualified |
| Processing Group 4A | qualified | qualified |
| Processing Group 5A | qualified | qualified |
| Processing Group 6A | outstanding | qualified |
| Processing Group 7A | qualified | qualified |
| Processing Group 8A | qualified | qualified |
| Processing Group 9A | outstanding | qualified |
| Processing Group 10A | outstanding | outstanding |
| Contrast Group 1A | unqualified | unqualified |

### Example 2

The formulation of the raw materials for the pectin gel was adjusted based on Processing Group 1A of Example 1, with the specific formulation numbers and formulation compositions shown in Table 6. Referring to the procedure for preparing pectin gels for Processing Group 1A of Example 1, the pectin gels were prepared separately for each Processing Group according to the formulations shown in Table 6. The sum of the content of PE and PI of the phospholipids was 60%, and the DE of the high-esterified pectin was 60%. In Processing Group 2B, citric acid: malic acid = 1:1 in terms of mass ratio; in Processing Group 3B, citric acid: lactic acid = 1:1 in terms of mass ratio; and in Processing Group 4B, maltitol syrup: xylitol = 1:1 and malic acid: lactic acid = 1:1 in terms of mass ratio.

**Table 6 Raw materials required for the preparation of pectin gels in each Processing Group of the present example**

| Components blending components | Processing Group 1B | | Processing Group 2B | | Processing Group 3B | | Processing Group 4B | |
|---|---|---|---|---|---|---|---|---|
| | Material | Content | Material | Content | Material | Content | Material | Content |
| | aqueous phase | | | | | oil phase | | |
| citric acid | maltitol syrup | glycerol | sodium citrate | water | high-esterified pectin | Rosemary Extract | phospholipid | arachidonic acid |
| 0.9% | 38% | 10% | 0.6% | remnant | 2% | 0.5% | 2% | 25% |
| citric acid malic acid | xylitol | glycerol | sodium citrate | water | high-esterified pectin | vitamin E acetate | phospholipid | evening primrose oil |
| 0.6% | 29% | 12% | 0.6% | remnant | 3% | 0.5% | 2.5% | 30% |
| citric acid lactic acid | sorbitol liquid | glycerol | sodium citrate | water | high-esterified pectin | tea polyphenols | phospholipid | linseed oil |
| 0.6% | 50% | 5% | 0.6% | remnant | 2.5% | 0.2% | 1.5% | 20% |
| malic acid lactic acid | maltitol syrup xylitol liquid | glycerol | sodium citrate | water | high-esterified pectin | ascorbic acid | phospholipid | caprylic/capric triglyceride (MCT) |
| 0.7% | 60% | 8% | 0.6% | remnant | 1.8% | 0.4% | 1% | 10% |

### Testing Example 2

### 1. Testing Objects

Pectin gels prepared from each Processing Group in Example 2.

### 2. Test Methods

(2) Texture test: The texture test was carried out using a texture meter, using the TPA method (texture profile analysis), which was a common method for testing texture characteristics in the field, and was also known as the secondary mastication test, in which the samples were compressed for a second time through the test probe, and then quantified by the force sensing element for the index. The samples were taken and placed in the middle of the test bench, the TPA method was selected, and the test key was activated for testing, in which the control samples were of uniform specifications and shapes, pancake shaped, 1.5g/piece, with a weight deviation of no more than 0.1g. 20 samples were tested in parallel in each group, and the average value was taken as the result of the texture test.

When the test results of hardness ≥500g and resilience ≥0.24, it was recorded as qualified and denoted by "O"; when the test results of hardness <500g and resilience <0.24, it was recorded as unqualified and denoted by "X".

(2) Storage stability: placing the test object under the environmental conditions at 50 °C and 80% relative humidity for 24h and observing the deformation of the test object, in which the presence of floppy deformation was recorded as unqualified, and it was denoted by "X"; and the absence of obvious floppy deformation was recorded as qualified, and it was denoted by "O".

### 3. Test results and analysis

The results of the present Testing Example are shown in Table 7, and it was confirmed by Processing Groups 1B-4B that a reasonably varied type of fat-soluble physiologically active substance and antioxidant or modulation of the content within the range of the formulation still resulted in a well-performing pectin gel.

**Table 7 Test results of Testing Example 2**

| Groups | Gel Performance | texture testing | Storage Stability |
|---|---|---|---|
| Processing Group 1B | O | O | O |
| Processing Group 2B | O | O | O |
| Processing Group 3B | O | O | O |
| Processing Group 4B | O | O | O |

### Example 3

### Processing Group 1C

### 1. Preparation of raw materials for pectin gels

The raw materials required for the preparation of pectin gels in Processing Group 1C of the present example are shown in Table 8. The sum of the content of the phospholipids PE and PI used was 60%.

**Table 8 Raw materials required for the preparation of pectin gels in Processing Group 1C of the present example**

| Components | | Material | Content in percentage/% |
|---|---|---|---|
| oil phase | fat-soluble physiologically active substances | DHA algae oil | 25 |
| | emulsifier | phospholipid | 0.66 |
| | antioxidant | Rosemary Extract | 0.5 |
| aqueous phase | gelling agent | high-esterified pectin (DE=60%) | 2 |
| | emulsifier | modified starch | 0.66 |
| | | gum Arabic | 0.66 |
| | / | glycerol | 10 |
| | | maltitol syrup | 38 |
| | / | water | remnant |
| blending components | acidity regulator | citric acid | 0.7 |
| | | sodium citrate | 0.1 |

### 2. Preparation method

Preparation of an aqueous phase: weighing the raw materials according to Table 8, adding the gelling agent, modified starch, and gum Arabic into the water, adding subsequently glycerol and maltitol syrup thereto, keeping the temperature of the reaction system above 90°C, controlling the pH at 4.1, holding the temperature at 85°C, and obtaining the aqueous phase.

Preparation of an oil phase: weighing the formulated fat-soluble physiological active substances, phospholipids, and antioxidants, mixing them well, and filling with nitrogen pending use;
Emulsifying homogenization: in terms of a mass ratio of the oil phase: the aqueous phase = 1:3, adding the oil phase to the aqueous phase and mixing homogeneously, emulsifying and homogenizing at 80 °C or above;
Blending: adding acidity regulator to the emulsified homogenized liquid, keeping the temperature of the reaction system above 60°C, controlling the pH at 3.5, filling with nitrogen pending use, and using the resulting liquid as a molding liquid;
Molding: pouring the molding liquid onto the blister, sealing and solidifying to obtain the pectin gel, in which the pH of the pectin gel was 3.5.

Different Processing Groups were set up by using the mass ratio between the gelling agent and the emulsifier in the composition of the formulations shown in Table 8 as a variable, and the variables corresponding to each Processing Group and the Contrasting Groups are shown in Table 9, in which the total mass content of the gelling agent and the emulsifier was kept unchanged and the mass ratio of phospholipids, modified starch, and gum Arabic was kept at 1:1:1. Except for the above differences, the procedure for preparing the pectin gels in each Processing Group was strictly consistent with that of Processing Group 1C of Example 3.

**Table 9 Variables in Processing Group 1C-4C and Contrast Group 1C**

| Groups | gelling agent: emulsifier | mass content of gelling agent | mass content of emulsifier |
|---|---|---|---|
| Processing Group 1C | 1:1 | 2% | 2% |
| Processing Group 2C | 3:1 | 3% | 1% |
| Processing Group 3C | 6:1 | 3.43% | 0.57% |
| Processing Group 4C | 12:1 | 3.7% | 0.3% |
| Contrast Group 1C | 1:6 | 0.57% | 3.43% |

Different Processing Groups were set up using the pH of the pectin gel as a variable, and the corresponding variables for each Processing Group are shown in Table 10. The pH of the pectin gels was achieved by regulating the pH adjusters added during preparation. Except for the above differences, the procedure for preparing the pectin gels in each Processing Group was strictly consistent with that of Processing Group 1C of Example 3.

**Table 10 Variables in Processing Group 5C-7C**

| Groups | pH of the pectin gel |
|---|---|
| Processing Group 1C | 3.5 |
| Processing Group 5C | 3.8 |
| Processing Group 6C | 3.0 |
| Processing Group 7C | 2.6 |

The mass ratio of modified starch to gum Arabic in the composition of the formulations exhibited in Table 8 was used as a variable to set up different Processing Groups, and the corresponding variables for each Processing Group are shown in Table 11. The total mass of modified starch and gum Arabic in pectin gels was kept unchanged, and the change in the mass ratio of modified starch to gum Arabic was achieved by increasing or decreasing the mass content of modified starch and gum Arabic, respectively. Except for the above differences, the procedure for preparing the pectin gels in each Processing Group was strictly consistent with that of Processing Group 1C of Example 3.

**Table 11 Variables in Processing Groups 11C-13C**

| Groups | gum Arabic modified starch |
|---|---|
| Processing Group 1C | 1:1 |
| Processing Group 8C | 0.5:1 |
| Processing Group 9C | 2:1 |
| Processing Group 10C | 4:1 |

### Testing Example 3

### 1. Testing Objects

Pectin gels prepared from each Processing Group and the Contrast Group in Example 3.

### 2. Test Methods

The test method of the present Testing Example was performed with reference to Testing Example 1.

### 3. Test results and analysis

The results of the present Testing Example are shown in Table 12, in which it was evident by comparing the data of the Processing Groups 1C-4C and the Contrast Group 1C, that the ratio of the gelling agent to the emulsifier affected the mechanical properties, texture, and the storage stability of the prepared pectin gels. In Processing Group 1C and Processing Groups 5C-7C, it was confirmed that the pH of the pectin gels likewise affected the mechanical properties, texture, and storage stability of the prepared pectin gels.

In Processing Group 1C, and Processing Groups 8C-10C, it was confirmed that the change in the mass ratio of modified starch to gum Arabic likewise affected the mechanical properties, texture, and storage stability of the prepared pectin gels. It was due to the gelatinization and viscosity-enhancing properties of modified starch, which affected the emulsification effect in the reaction system and the viscosity of the slurry, which consequently affected the gelling speed and gel stability of the pectin gels.

Therefore, an improvement in the texture and storage stability of a pectin gel containing a fat-soluble substance was achieved in the present disclosure by regulating the ratio of a gelling agent to an emulsifier in the pectin gel, the pH of the pectin gel, and the change in the mass ratio of modified starch to gum Arabic.

**Table 12 Test results of Testing Example 3**

| Groups | texture testing | Storage Stability |
|---|---|---|
| Processing Group 1C | outstanding | outstanding |
| Processing Group 2C | outstanding | qualified |
| Processing Group 3C | outstanding | qualified |
| Processing Group 4C | qualified | qualified |
| Processing Group 5C | qualified | qualified |
| Processing Group 6C | outstanding | qualified |

| | | |
|---|---|---|
| Processing Group 7C | qualified | qualified |
| Processing Group 8C | qualified | qualified |
| Processing Group 9C | qualified | outstanding |
| Processing Group 10C | qualified | qualified |
| Processing Group 1C | unqualified | unqualified |

### Example 4

The formulation of the raw materials for the pectin gel was adjusted based on Processing Group 1C of Example 3, with the specific formulation numbers and formulation compositions shown in Table 13. Referring to the procedure for preparing pectin gels for Processing Group 1C of Example 3, the pectin gels were prepared separately for each Processing Group according to the formulations shown in Table 13. The sum of the content of PE and PI of the phospholipids was 60%, and the DE of the lhigh-esterified pectin was 60%. In Processing Group 3D, citric acid: malic acid = 1:1 in terms of the mass ratio; and in Processing Group 4D, maltitol syrup: xylitol = 1:1 and citric acid: lactic acid = 1:1 in terms of the mass ratio.

**Table 13 Raw materials required for the preparation of pectin gels for each Processing Group of the present example**

| oil phase | | | | | | Components | |
|---|---|---|---|---|---|---|---|
| Rosemary Extract | | phospholipid | | safflower seed oil | | Material | Processing Group 1D |
| 0.5% | | 0.66% | | 25% | | Content | |
| Rosemary Extract | | phospholipid | | silybum marianum seed oil | | Material | Processing Group 2D |
| 0.5% | | 1% | | 35% | | Content | |
| Rosemary Extract | | phospholipid | | acer truncatum bunge seed oil | | Material | Processing Group 3D |
| 0.3% | | 0.8% | | 15% | | Content | |
| Rosemary Extract | | phospholipid | | γ-linolenic acid oil | | Material | Processing Group 4D |
| 0.2% | | 0.5% | | 10% | | Content | |

| blending components | | aqueous phase | | | | | |
|---|---|---|---|---|---|---|---|
| sodium citrate | citric acid | maltitol syrup | glycerol | gum Arabic | modified starch | water | high-esterified pectin |
| 0.1% | 0.7% | 38% | 10% | 0.66% | 0.66% | remnant | 2% |
| sodium citrate | malic acid | xylitol liquid | glycerol | gum Arabic | modified starch | water | high-esterified pectin |
| 0.1% | 0.6% | 30% | 5% | 1% | 1% | remnant | 3% |
| sodium citrate | citric acid malic acid | sorbitol liquid | glycerol | gum Arabic | modified starch | water | high-esterified pectin |
| 0.1% | 0.5% | 48% | 12% | 0.8% | 0.8% | remnant | 2% |
| sodium citrate | citric acid lactic acid | maltitol syrup xylitol liquid | glycerol | gum Arabic | modified starch | water | high-esterified pectin |
| 0.2% | 0.5% | 56% | 11% | 0.5% | 0.5% | remnant | 1.8% |

### Testing Example 4

### 1. Testing Objects

Pectin gels prepared from each Processing Group in Example 4.

### 2. Test Methods

Reference to the method provided in Testing Example 2 was performed.

### 3. Test results and analysis

The results of the present Testing Example are shown in Table 14, and it was confirmed by Processing Groups 1D-4D that a reasonably varied type of fat-soluble physiologically active substance and antioxidant or modulation of the content within the range of the formulation still resulted in a well-performing pectin gel.

**Table 14 Test results of Testing Example 4**

| Groups | Gel Performance | texture testing | Storage Stability |
|---|---|---|---|
| Processing Group 1D | O | O | O |
| Processing Group 2D | O | O | O |
| Processing Group 3D | O | O | O |
| Processing Group 4D | O | O | O |

### Example 5

### Processing Group 1E

### 1. Preparation of raw materials for pectin gels

The raw materials required for the preparation of pectin gels in Process Group 1E in the present example are shown in Table 15, in which the content of acetyl groups of emulsified pectin was 20%.

**Table 15 Raw materials required for the preparation of pectin gels in Processing Group 1E of the present example**

| Components | | Material | Content in percentage/% |
|---|---|---|---|
| oil phase | fat-soluble physiologically active substances | DHA algae oil | 25 |
| | antioxidant | Rosemary Extract | 0.5 |
| aqueous phase | gelling agent | high-esterified pectin (DE=60%) | 3 |
| | emulsifier | Emulsified pectin (DE=55%) | 1 |
| | / | water | remnant |
| | / | sodium citrate | 0.6 |
| | / | glycerol | 10 |
| | | maltitol syrup | 40 |
| blending components | acidity regulator | citric acid | 0.8 |

### 2. Preparation method

Preparation of an aqueous phase: weighing the raw materials according to Table 15, adding the gelling agent and emulsified pectin into the water, adding subsequently glycerol, maltitol syrup, and sodium citrate thereto, keeping the temperature of the reaction system above 90°C, controlling the pH at 4.1, holding the temperature at 85°C, and obtaining the aqueous phase.

Preparation of an oil phase: weighing the formulated fat-soluble physiological active substances and antioxidants, mixing them well, and filling with nitrogen pending use;
Emulsifying homogenization: in terms of a mass ratio of the oil phase: the aqueous phase = 1:3, adding the oil phase to the aqueous phase and mixing homogeneously, emulsifying and homogenizing at 80 °C or above;
Blending: adding acidity regulator to the emulsified homogenized liquid, keeping the temperature of the reaction system above 60°C, controlling the pH at 3.5, filling with nitrogen pending use, and using the resulting liquid as a molding liquid;
Molding: pouring the molding liquid onto the blister, sealing, and solidifying to obtain the pectin gel, in which the pH of the pectin gel was 3.5.

Different Processing Groups were set up by using the mass ratio between the gelling agent and the emulsifier in the composition of the formulations shown in Table 15 as a variable, and the variables corresponding to each Processing Group and Contrast Group are shown in Table 16, in which the total mass content of the gelling agent and the emulsifier was kept unchanged. Except for the above differences, the procedure for preparing the pectin gels in each Processing Group was strictly consistent with that of Processing Group 1E of Example 5.

**Table 16 Variables in Processing Groups 1E-4E and Contrast group 1E**

| Groups | gelling agent: emulsifier | mass content of gelling agent | mass content of emulsifier |
|---|---|---|---|
| Processing Group 1E | 3:1 | 3% | 1% |
| Processing Group 2E | 1:1 | 2% | 2% |
| Processing Group 3E | 5:1 | 3.4% | 0.6% |
| Processing Group 4E | 10:1 | 3.64% | 0.36% |
| Contrast Group 1E | 1:5 | 0.6% | 3.4% |

Different Processing Groups were set up using the content of lipophilic groups (acetyl groups) of the emulsified pectin used for the formulations shown in Table 15 as a variable, and the corresponding variables for each Processing Group are shown in Table 17. Except for the above differences, the procedure for preparing the pectin gels in each Processing Group was strictly consistent with that of Processing Group 1E of Example 5.

**Table 17 Variables in Processing Groups 5E-6E**

| Groups | acetyl content of emulsified pectin |
|---|---|
| Processing Group 1E | 20% |
| Processing Group 5E' | 15% |
| Processing Group 6E' | 30% |

Different Processing Groups were set up using the pH of the pectin gel as a variable, and the corresponding variables for each Processing Group are shown in Table 18. The pH of the pectin gel was achieved by regulating the pH adjuster added during preparation, and the change in the content of the pH adjuster was achieved by increasing or decreasing the mass content of the sugar alcohol. Except for the above differences, the procedure for preparing the pectin gels in each Processing Group was strictly consistent with that of Processing Group 1E of Example 5.

**Table 18 Variables in Processing Groups 7E-9E**

| Groups | pH of the pectin gel |
|---|---|
| Processing Group 1E | 3.5 |
| Processing Group 7E | 3.8 |
| Processing Group 8E | 3.0 |
| Processing Group 9E | 2.6 |

Different Processing Groups were set up using the mass ratio of glycerol to sugar alcohols in the composition of the formulations shown in Table 15 as a variable, and the corresponding variables for each Processing Group are shown in Table 19. The total mass of glycerol and sugar alcohol in the pectin gel remains constant, and the change in the mass ratio of glycerol to sugar alcohol was achieved by increasing or decreasing the mass content of glycerol and sugar alcohol, respectively. Except for the above differences, the procedure for preparing the pectin gels in each Processing Group was strictly consistent with that of Processing Group 1E of Example 5.

**Table 19 Variables in Processing Groups 10E-11E**

| Groups | glycerol: sugar alcohol |
|---|---|
| Processing Group 1E | 10:40 |
| Processing Group 10E | 3:35 |
| Processing Group 11E | 3:70 |

### Testing Example 5

### 1. Testing Objects

Pectin gels prepared from each Processing Group and the Contrast Group in Example 5.

### 2. Test Methods

The test method of the present Testing Example was performed with reference to Testing Example 1.

### 3. Test results and analysis

The results of the present Testing Example are shown in Table 20, in which it was evident by comparing the data of the Processing Groups 1E-4E and the Contrast Group 1E, that the ratio of the gelling agent to the emulsifier affected the mechanical properties, texture, and the storage stability of the prepared pectin gels. In Processing Group 1E and Processing Groups 5E-6E, it was then confirmed that although emulsified pectin was unable to be gelatinized, the acetyl content of emulsified pectin affected the emulsifying properties of emulsified pectin, thereby affecting the texture of the pectin gels.

In Processing Group 1E and Processing Groups 7E-9E, it was confirmed that the pH of the pectin gels likewise affected the mechanical properties, texture, and storage stability of the prepared pectin gels.

In Processing Group 1E and Processing Group 10E-11E, it was then confirmed that the mass ratio of glycerol to sugar alcohols affected the range of sugar levels in the pectin gels, thereby affecting the gel properties. Besides this, it also improves the moisturizing effect of the pectin gel. Therefore, a texture was optimized in the present disclosure by further restricting the mass ratio of glycerol to sugar alcohols.

Therefore, an improvement in the texture and storage stability of a pectin gel containing a fat-soluble substance was achieved in the present disclosure by regulating the ratio of a gelling agent to an emulsifier in the pectin gel, the pH of the pectin gel, the acetyl content of emulsified pectin, and the change to the mass ration of glycerol to sugar alcohols.

**Table 20 Test results of Testing Example 5**

| Groups | texture testing | Storage Stability |
|---|---|---|
| Processing Group 1E | outstanding | outstanding |
| Processing Group 2E | qualified | qualified |
| Processing Group 3E | outstanding | qualified |
| Processing Group 4E | qualified | qualified |
| Processing Group 5E | outstanding | outstanding |
| Processing Group 6E | qualified | qualified |
| Processing Group 7E | qualified | qualified |
| Processing Group 8E | outstanding | outstanding |
| Processing Group 9E | qualified | qualified |
| Processing Group 10E | outstanding | qualified |

| | | |
|---|---|---|
| Processing Group 11E | outstanding | qualified |
| Processing Group 1E | unqualified | unqualified |

### Example 6

The formulation of the raw materials for the pectin gel was adjusted based on Processing Group 1E of Example 5, with the specific formulation numbers and formulation compositions shown in Table 21. Referring to the procedure for preparing pectin gels for Processing Group 1E of Example 5, the pectin gels were prepared separately for each Processing Group according to the formulations shown in Table 21. The DE of high-esterified pectin was 60% and that of emulsified pectin was 55%; in Processing Group 2F, citric acid: lactic acid = 1:1 in terms of the mass ratio; in Processing Group 3F, citric acid: malic acid = 1:1 in terms of the mass ratio; and in Processing Group 4F, sorbitol solution: xylitol = 1:1 and lactic acid : malic acid = 1:1 in terms of the mass ratio.

**Table 12 Raw materials required for the preparation of pectin gels for each Processing Group of the present example**

| aqueous phase | | | | oil phase | | Components | |
|---|---|---|---|---|---|---|---|
| glycerol | sodium citrate | emulsified pectin | high-esterified pectin | vitamin E acetate | walnut oil | Material | Processing Group 1F |
| 10% | 0.6% | 1% | 3% | 0.5% | 25% | Content | |
| glycerol | sodium citrate | emulsified pectin | high-esterified pectin | vitamin E acetate | fish oil | Material | Processing Group 2F |
| 3% | 0.6% | 1.2% | 3% | 0.5% | 30% | Content | |
| glycerol | sodium citrate | emulsified pectin | high-esterified pectin | vitamin E acetate | coenzyme Q10 | Material | Processing Group 3F |
| 7% | 0.6% | 0.8% | 2.5% | 0.1% | 15% | Content | |
| glycerol | sodium citrate | emulsified pectin | high-esterified pectin | vitamin E acetate | Rice bran fatty alcohol | Material | Processing Group 4F |
| 12% | 0.6% | 0.5% | 2% | 0.2% | 10% | Content | |

| | blending components | | | | | | |
|---|---|---|---|---|---|---|---|
| | citric acid | water | | maltitol syrup | | | |
| | 0.8% | remnant | | 40% | | | |
| | citric acid lactic acid | water | | xylitol liquid | | | |
| | 0.6% | remnant | | 40% | | | |
| | citric acid malic acid | water | | sorbitol liquid | | | |
| | 0.6% | remnant | | 55% | | | |
| | lactic acid malic acid | water | | sorbitol liquid xylitol liquid | | | |
| | 0.7% | remnant | | 56% | | | |

### Testing Example 4

### 1. Testing Objects

Pectin gels prepared from each Processing Group in Example 4.

### 2. Test Methods

Reference to the method provided in Testing Example 2 was performed.

### 3. Test results and analysis

The results of the present Testing Example are shown in Table 22, and it was confirmed by Processing Groups 1F-4F that a reasonably varied type of fat-soluble physiologically active substance or modulation of the content within the range of the formulation still resulted in a well-performing pectin gel.

**Table 22 Test results of Testing Example 6**

| Groups | Gel Performance | texture testing | Storage Stability |
|---|---|---|---|
| Processing Group 1F | O | O | O |
| Processing Group 2F | O | O | O |
| Processing Group 3F | O | O | O |
| Processing Group 4F | O | O | O |

### Example 7

### Processing Group 1G

### 1. Preparation of raw materials for pectin gels

The raw materials required for the preparation of pectin gels in Processing Group 1G of the Present example are shown in Table 23. The sum of the content of the phospholipids PE and PI used was 60%.

**Table 23 Raw materials required for the preparation of pectin gels in Processing Group 1G of the present example**

| Components | | Material | Content in percentage/% |
|---|---|---|---|
| oil phase | fat-soluble physiologically active substances | DHA algae oil | 25 |
| | emulsifier | phospholipid | 1.5 |
| | antioxidant | Rosemary Extract | 0.5 |
| aqueous phase | gelling agent | low-esterified pectin (DE=30%) | 3 |
| | / | sodium citrate | 0.4 |
| | / | water | remnant |
| | / | glycerol | 3 |
| | | maltitol syrup | 44 |
| blending components | acidity regulator | citric acid | 3 |
| | | sodium citrate | 1 |
| | calcium salt | tricalcium phosphate | 0.2 |
| | amino acid | γ-Aminobutyric acid | 2 |

### 2. Preparation method

Preparation of an aqueous phase: weighing the raw materials according to Table 23, adding the gelling agent into the water, adding subsequently glycerol, maltitol syrup, and sodium citrate thereto, keeping the temperature of the reaction system above 90°C, controlling the pH at 4.2, holding the temperature at 85°C, and obtaining the aqueous phase.

Preparation of an oil phase: weighing the formulated fat-soluble physiological active substances, phospholipids, and antioxidants, mixing them well, and filling with nitrogen pending use;
Emulsifying homogenization: in terms of a mass ratio of the oil phase: the aqueous phase = 1:3, adding the oil phase to the aqueous phase and mixing homogeneously, emulsifying and homogenizing at 80 °C or above;
Blending: adding calcium salt, acidity regulator, amino acid into the emulsified homogenized liquid, keeping the temperature of the reaction system above 60°C, controlling the pH at 4.1, filling with nitrogen and pending for use, and taking the liquid obtained as a molding liquid;
Molding: pouring the molding liquid onto the blister, sealing, and solidifying to obtain the pectin gel, in which the pH of the pectin gel was 4.1.

Different Processing Groups were set up by using the mass ratio between the gelling agent and the emulsifier in the composition of the formulations shown in Table 23 as a variable, and the variables corresponding to each Processing Group and Contrast Group are shown in Table 24, in which the total mass content of the gelling agent and the emulsifier was kept unchanged. Except for the above differences, the procedure for preparing the pectin gels in each Processing Group was strictly consistent with that of Processing Group 1G of Example 7.

**Table 24 Variables in Processing Groups 2G-4G**

| Groups | gelling agent: emulsifier | mass content of gelling agent | mass content of emulsifier |
|---|---|---|---|
| Processing Group 1G | 2:1 | 3% | 1.5% |
| Processing Group 2G | 1:1 | 2.25% | 2.25% |
| Processing Group 3G | 5:1 | 3.25% | 0.75% |
| Processing Group 4G | 10:1 | 3.6% | 0.4% |
| Contrast Group 1G | 1:6 | 0.57% | 3.43% |

Different Processing Groups were set up using the pH of the pectin gel as a variable, and the corresponding variables for each Processing Group are shown in Table 25. The pH of the pectin gel was achieved by regulating the pH adjuster added during preparation, and the change in the content of the pH adjuster was achieved by increasing or decreasing the mass content of the sugar alcohol. Except for the above differences, the procedure for preparing the pectin gels in each Processing Group was strictly consistent with that of Processing Group 1G of Example 7.

**Table 25 Variables in Processing Groups 5G-6G**

| Groups | pH of the pectin gel |
|---|---|
| Processing Group 1G | 4 |
| Processing Group 5G | 5 |
| Processing Group 6G | 2.6 |

Different Processing Groups were set up using the content ratio of emulsifier to calcium ions used for the formulation shown in Table 23 as a variable, and the corresponding variables for each Processing Group are shown in Table 26, in which the mass percentage of the emulsifier was a, the mass percentage of the calcium ions was b, and the content ratio of emulsifier to calcium ions was a/b. The content of emulsifiers and calcium ions was achieved by increasing or decreasing the mass content of sugar alcohols. Except for the above differences, the procedure for preparing the pectin gels in each Processing Group was strictly consistent with that of Processing Group 1G of Example 7. In Processing Group 1G, the calcium salt used was tricalcium phosphate (dosage 0.2%), which had a content of 34% calcium ions, which corresponded to a content of 0.068% calcium ions in the pectin gel.

**Table 26 Variables in Processing Group 1G and Processing Group 7G-8G**

| Groups | a | b | a/b |
|---|---|---|---|
| Processing Group 1G | 31.5% | 0.068% | 22 |
| Processing Group 7G | 4.25% | 0.034% | 125 |
| Processing Group 8G | 6.8% | 0.027% | 250 |

Different Processing Groups were set up using the type of calcium salts used in the composition of the formulations shown in Table 23 as a variable, and the corresponding variables for each Processing Group are shown in Table 27. The total mass of calcium salts in the pectin gel remained unchanged and the change in the content of calcium ions changed due to the type of calcium salts, as shown in Table 23. Except for the above differences, the procedure for preparing the pectin gels in each Processing Group was strictly consistent with that of Processing Group 1G of Example 7.

**Table 27 Variables in Processing Groups 12G-14G**

| Groups | Types of calcium salts | Content of calcium ions |
|---|---|---|
| Processing Group 1G | tricalcium phosphate | 0.068% |
| Processing Group 9G | calcium lactate | 0.061% |
| Processing Group 10G | calcium citrate | 0.042% |
| Processing Group 11G | calcium hydrogen phosphate | 0.047% |

### Testing Example 7

### 1. Testing Objects

Pectin gels prepared from each Processing Group and the Contrast Group in Example 7.

### 2. Test Methods

The test method of the present Testing Example was performed with reference to Testing Example 1.

### 3. Test results and analysis

The results of the present Testing Example are shown in Table 28, in which it was evident by comparing the data of the Processing Groups 1G-4G and the Contrast Group 1G, that the ratio of the gelling agent to the emulsifier affected the mechanical properties, texture, and the storage stability of the prepared pectin gels. In Processing Group 1G and Processing Groups 5G-7G, it was confirmed that the pH of the pectin gels likewise affected the mechanical properties, texture, and storage stability of the prepared pectin gels.

In Processing Group 1G and Processing Groups 7G-8G, it was then demonstrated that the formation of organized cross-linking structure of low-esterified pectin was enhanced by regulating the ratio of the content of emulsifier to calcium ions as a/b, which consequently improved the gel properties and texture of the pectin gels. In Processing Group 1G and Processing Groups 9G-11G, it was then confirmed that the content of calcium ions varied with the change in the type of calcium salt selected, which consequently affected the properties of the prepared pectin gels.

Therefore, an improvement in the texture and storage stability of a pectin gel containing a fat-soluble substance was achieved in the present disclosure by regulating the ratio of a gelling agent to an emulsifier in the pectin gel, the pH of the pectin gel, content ratio of emulsifier to calcium ions, and change in type of calcium salts.

**Table 28 Test results of Testing Example 7**

| Groups | texture testing | Storage Stability |
|---|---|---|
| Processing Group 1G | outstanding | outstanding |
| Processing Group 2G | qualified | outstanding |
| Processing Group 3G | outstanding | outstanding |
| Processing Group 4G | qualified | qualified |
| Processing Group 5G | qualified | qualified |
| Processing Group 6G | qualified | qualified |
| Processing Group 7G | qualified | outstanding |
| Processing Group 8G | qualified | qualified |
| Processing Group 9G | outstanding | outstanding |
| Processing Group 10G | qualified | outstanding |
| Processing Group 11G | qualified | outstanding |
| Contrast Group 1G | unqualified | unqualified |

### Example 8

The formulation of the raw materials for the pectin gel was adjusted based on Processing Group 1G of Example 7, with the specific formulation numbers and formulation compositions shown in Table 29. Referring to the procedure for preparing pectin gels for Processing Group 1G of Example 7, the pectin gels were prepared separately for each Processing Group according to the formulations shown in Table 29. The sum of the content of PE and PI of the phospholipids used was 60%, and the DE of low-esterified pectin was 30%; in Processing Group 3H, the maltitol syrup: erythritol = 3:1 in terms of the mass ratio; in Processing Group 4H, the sorbitol solution: erythritol = 3:1 in terms of the mass ratio.

**Table 29 Raw materials required for the preparation of pectin gels for each Processing Group of the present example**

| aqueous phase | | | | | oil phase | | | Components | |
|---|---|---|---|---|---|---|---|---|---|
| maltitol syrup | glycerol | sodium citrate | water | low-esterified pectin | L-Theanine | phospholipid | DHA algae oil | Material | Processing Group 1H |
| 44% | 3% | 0.4% | remnant | 3% | 0.5% | 1.5% | 25% | Content | |
| sorbitol liquid | glycerol | sodium citrate | water | low-esterified pectin | L-Theanine | phospholipid | pumpkin seed oil | Material | Processing Group 2H |
| 37% | 3% | 0.4% | remnant | 3.5% | 0.5% | 3% | 30% | Content | |
| maltitol syrup erythritol | glycerol | sodium citrate | water | low-esterified pectin | L-Theanine | phospholipid | Borage Oil | Material | Processing Group 3H |
| 48% | 5% | 0.4% | remnant | 2.8% | 0.3% | 2% | 15% | Content | |
| sorbitol liquid erythritol | glycerol | sodium citrate | water | low-esterified pectin | L-Theanine | phospholipid | MCT | Material | Processing Group 4H |
| 48% | 8% | 0.4% | remnant | 2.3% | 0.2% | 1% | 10% | Content | |
| blending components | citric acid | 3% | citric acid | 3.5% | citric acid | 5% | citric acid | 5% | |
| | sodium citrate | 1% | sodium citrate | 1% | sodium citrate | 5% | sodium citrate | 1.5% | |
| | tricalcium phosphate | 0.2% | calcium lactate | 0.1% | calcium citrate | 0.2% | calcium hydrogen | 0.2% | |
| | γ-Aminobutyric acid | 2% | γ-Aminobutyric acid | 1.6% | creatine | 1.6% | γ-Aminobutyric acid | 3.3% | |

### Testing Example 8

### 1. Testing Objects

Pectin gels prepared from each Processing Group in Example 8.

### 2. Test Methods

Reference to the method provided in Testing Example 2 was performed.

### 3. Test results and analysis

The results of the present Testing Example are shown in Table 30, and it was confirmed by Processing Groups 1H-4H that a reasonably varied type of antioxidant and amino acid or modulation of the content within the range of the formulation still resulted in a well-performing pectin gel.

**Table 30 Test results of Testing Example 8**

| Groups | Gel Performance | texture testing | Storage Stability |
|---|---|---|---|
| Processing Group 1H | O | O | O |
| Processing Group 2H | O | O | O |
| Processing Group 3H | O | O | O |
| Processing Group 4H | O | O | O |

The above examples are only used to illustrate the technical solution of the present disclosure rather than to limit the scope of protection of the present disclosure, although the present disclosure has been described in detail with reference to the preferred embodiments, those of ordinary skill in the art should understand that modifications or equivalent substitutions may be made to the technical solution of the present disclosure, without departing from the substance and scope of the technical solution of the present disclosure.

## Claims

1. A pectin gel, comprising a gelling agent, an emulsifier and a fat-soluble substance, wherein the gelling agent comprises pectin with a degree of esterification of not less than 20%, and, in terms of a mass ratio, the gelling agent:the emulsifier=(0.2-30):1.

2. The pectin gel according to claim 1, wherein the fat-soluble substance comprises a fat-soluble physiologically active substance, and a mass percentage of the fat-soluble physiologically active substance in the pectin gel is 10%-70%.

3. The pectin gel according to claim 1, wherein an HLB value of the emulsifier is 7-18, and the emulsifier comprises at least one of a phospholipid emulsifier, modified starch, gum, emulsified pectin, cholesterol, lanolin, saponin, and polysaccharide emulsifiers.

4. The pectin gel according to claim 3, wherein a content of lipophilic groups in the emulsified pectin is 5%-35%, and phospholipid emulsifiers comprise phospholipids, and the phospholipids comprise phosphatidylethanolamine and phosphatidylinositol.

5. The pectin gel according to claim 3, wherein the emulsifier comprises a proteinaceous emulsifier, and an isoelectric point of the proteinaceous emulsifier is greater than or equal to 3.

6. The pectin gel according to claim 1, wherein the gelling agent comprises high-esterified pectin, the high-esterified pectin refers to pectin with a degree of esterification greater than 50%, a content of the high-esterified pectin in the pectin gel is 1.5%-3.5% in terms of mass percentage, and a pH value of the pectin gel is 2.6-3.8, and a content of soluble solids of the pectin gel is greater than or equal to 55% in terms of mass percentage.

7. The pectin gel according to claim 6, wherein the pectin gel contains glycerol and sugar alcohol, and a mass of the glycerol:a mass of the sugar alcohol=(1-15):(25-70).

8. The pectin gel according to claim 6, wherein the emulsifier comprises phospholipids, and a content of the phospholipids in the pectin gel is 0.1%-5% in terms of mass percentage.

9. The pectin gel according to claim 6, wherein the emulsifier comprises modified starch, and a content of the modified starch in the pectin gel is 0. 1%-4% in terms of mass percentage.

10. The pectin gel according to claim 6, wherein the emulsifier comprises gum Arabic, and a mass of the gum Arabic:a mass of the high-esterified pectin=(1-2):(1.5-8).

11. The pectin gel according to claim 1, wherein the pectin comprises low-esterified pectin, the low-esterified pectin refers to pectin with a degree of esterification of 20%-50%, a content of the low-esterified pectin in the pectin gel is 1.5%-4% in terms of mass percentage, and a pH value of the pectin gel is 2.6-5, and a content of soluble solids of the pectin gel is greater than or equal to 10% in terms of mass percentage .

12. The pectin gel according to claim 11, wherein the low-esterified pectin comprises amide pectin, and a degree of amidation of the amide pectin is 12%-25%.

13. The pectin gel according to claim 11, further comprising metallic ions, and a content of the metallic ions in the pectin gel is 0.02-0.24%, in terms of mass percentage, wherein the metallic ions comprise the calcium ions, a mass percentage of the emulsifier in the pectin gel is a, a mass percentage of the calcium ions in the pectin gel is b, and the pectin gel satisfies 0.4≤a/b≤250 .

14. The pectin gel according to claim 11, further comprising amino acid, and a content of the amino acid in the pectin gel is no more than 10% in terms of mass percentage.

15. A gel product, comprising a pectin gel as claimed in any one of claims 1-14, wherein a dosage form of the gel product comprises at least one of a gummy candy, an oil gel, a gel emulsion, a softgel capsule especially a chewable softgel capsule, a crystal ball, a popping bead, and a drink.
